# EUROPÄISCHE PATENTSCHRIFT

(11) **EP 1 714 672 B1**
(45) Veröffentlichungstag und Bekanntmachung des Hinweises auf die Patenterteilung: **31.10.2007**
(21) Anmeldenummer: 06006091.0
(22) Anmeldetag: 24.03.2006
(51) Int. Cl.: A61N 1/05, H01R 31/06

(54) **Kontaktverbindungs-Adapter zur Herstellung eines zeitweisen elektrischen Kontaktes zwischen zwei Steckern**
Contact connecting adapter for the creation of an occasional electrical contact between two plugs
Adaptateur de relation de contact visant la création d'un contact électrique occasionnel entre deux connecteurs

(30) Priorität: 22.04.2005 DE 102005018806
(43) Veröffentlichungstag der Anmeldung: 25.10.2006
(73) Patentinhaber: BIOTRONIK CRM Patent AG, 6341 Baar (CH)
(72) Erfinder: Landgraf, Tassilo, 12277 Berlin (DE)
(74) Vertreter: Lindner-Vogt, Karin L.

(56) Entgegenhaltungen:
- US-A- 5 782 892
- US-A1- 2003 120 327
- US-B1- 6 343 233
- ANONYMOUS: "Cable connector for electrical measurements" RESEARCH DISCLOSURE, MASON PUBLICATIONS, HAMPSHIRE, GB, Bd. 395, Nr. 11, März 1997 (1997-03), XP007121588 ISSN: 0374-4353

## Beschreibung

Die Erfindung betrifft einen Kontaktverbindungs-Adapter zur Herstellung eines zeitweisen elektrischen Kontaktes zwischen einem ersten Stecker, insbesondere einem Norm-Anschluss-Stecker einer Herzschrittmacher-Elektrode, und mindestens einem zweiten Stecker, insbesondere einem üblichen Laborstecker zur zeitweisen Verbindung der Elektrode mit einem Reizschwellenanalysator.

Der Hintergrund der Erfindung liegt in der Vorgehensweise beim Implantieren eines Herzschrittmachers, Defibrillators oder ähnlichen kardiologischen Gerätes, dessen elektrophysiologischen Simulationsimpulse durch entsprechend im oder am Herzen positionierte Elektroden abgegeben werden. Beim Implantationsvorgang werden üblicherweise die Elektroden über das Gefäßsystem des Patienten mithilfe eines in die Elektrode eingeschobenen Mandrins gesteuert vorgeschoben. Dieser Mandrin läuft dabei durch den proximalen Anschlussstecker der Elektrode koaxial hindurch. Bei diesem Stecker handelt es sich in der Regel um einen für die medizinische Anwendung genormten Stecker etwa der Bezeichnung IS-1 / IS-4 / DF-1 etc., der unipolar oder bipolar ausgeführt sein kann.

Nach dem Setzen einer Elektrode müssen deren Sitz und Reizimpuls-Abgabeverhalten analysiert werden, wofür ein so genannter Reizschwellenanalysator zuständig ist. Dieses Gerät simuliert den ansonsten über den Elektroden-Anschlussstecker angekoppelten Herzschrittmacher und muss dazu elektrisch mit dem Elektrodenstecker verbunden werden. Dies kann jedoch nicht durch einfaches Einstecken des Steckers in das Gerät erfolgen, da zum einen der Elektrodenstecker steril gehalten werden muss, zum anderen soll der Mandrin für den vorstehenden Test noch nicht aus der Elektrode gezogen werden, da gegebenenfalls eine Neupositionierung der Elektrode mithilfe des Steuermandrins notwendig wird. Da das Mandrinende durch den Stecker geführt ist, könnte der Stecker ohnehin nicht in eine Gerätebuchse eingeführt werden.

Die DE 198 10 262 A1 (= US 6,708,067 B1) offenbart eine Testkabelanordnung, bei der auf einem kartonähnlichen Träger der Elektrodenstecker mit einem Anschlusspol in einer auf dem Träger befestigten Klammerklemme fixierbar ist. Der zweite Pol, in der Regel ein Elektrodenanschlussring, wird über eine Krokodilklemme kontaktiert, die gleichzeitig eine weitere mechanische Verbindung zwischen den kartenähnlichen Träger und dem Elektrodenstecker herstellt. Klammerklemme und Krokodilklemme sind über dünne Litzen elektrisch angeschlossen, die über eine entsprechende Steckvorrichtung in eine passende Buchse des Testgerätes eingesteckt werden können.

Diese vorbekannte Lösung hat einerseits einen provisorischen Charakter, andererseits sind für die Herstellung des Adapters mehrere Schritte notwendig, nämlich das Zuschneiden des kartenförmigen Trägers sowie das Verdrahten und Anbringen der Klammer- und Krokodilklemme. Die Handhabung dieses Testadapters ist umständlich und die Kontaktgabe insbesondere zur Krokodilklemme in ihrer Zuverlässigkeit verbesserungsbedürftig.

Das Dokument US-A-5 782 892 offenbart einen Kontaktverbindungs-Adapter gemäß Präambel des Anspruchs 1.

Der Erfindung liegt die Aufgabe zugrunde, einen Kontaktverbindungs-Adapter der eingangs genannten Art anzugeben, der mit deutlich geringerem Herstellungsaufwand fertigbar, extrem einfach zu handhaben ist und dabei eine hohe Kontaktzuverlässigkeit zwischen den zu verbindenden elektrischen Teilen bietet.

Diese Aufgabe wird durch die Merkmale des Anspruches 1 gelöst.

Erkennbar benötigt der Grundkörper selbst also keinerlei Kontaktelemente zur Herstellung eines elektrischen Kontaktes. Diese werden allein über die eingesteckten Stecker direkt miteinander hergestellt. Damit kann der Kontaktverbindungs-Adapter aus einem einheitlichen Werkstoff, wie beispielsweise spritzfähigem Kunststoff, hergestellt sein. Der Adapter zeichnet sich damit auch durch eine besonders gute Sterilisierbarkeit aus.

In den Unteransprüchen sind bevorzugte Weiterbildungen des Kontaktverbindungs-Adapters angegeben, deren Merkmale, Einzelheiten und Vorteile der nachfolgenden Beschreibung eines Ausführungsbeispiels des Erfindungsgegenstandes anhand der beigefügten Zeichnungen deutlich werden. Es zeigen:
- Fig.1: einen Horizontalschnitt eines Kontaktverbindungs-Adapters gemäß der Schnittlinie I-I nach Fig. 2 mit zu verbindenden Steckern vor deren Einschieben in den Adapter,
- Fig. 2: eine Seitenansicht des Adapters aus Pfeilrichtung II nach Fig. 1, und
- Fig. 3: einen Schnitt des Adapters gemäß der Schnittlinie III-III nach Fig. 2.

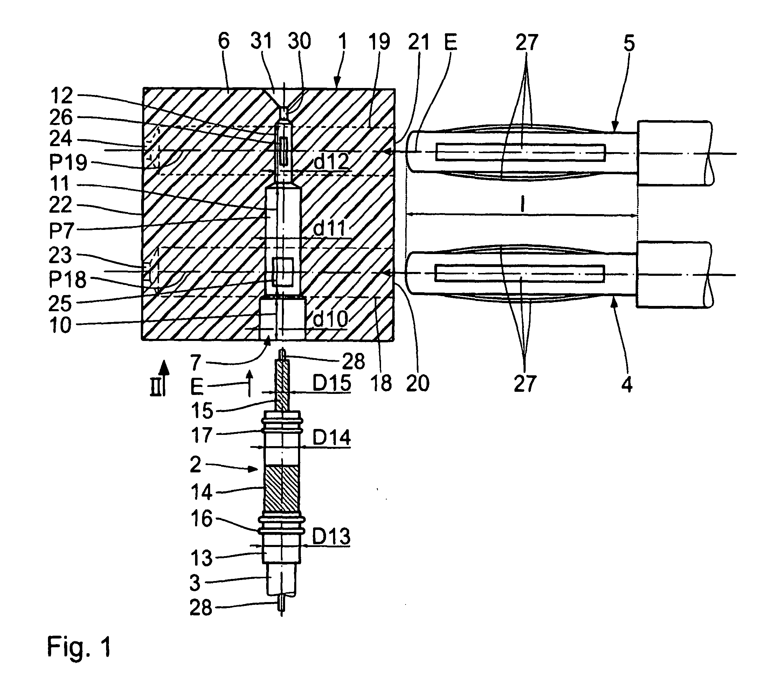

Der in den Zeichnungen dargestellte Kontaktverbindungs-Adapter 1 dient zur Herstellung eines zeitweisen elektrischen Kontaktes zwischen einerseits einem genormten IS-1 / IS-4 / DF-1-Anschlussstecker 2 einer in Fig. 1 angeschnitten dargestellten Herzelektrode 3 und andererseits zwei üblichen Laborsteckern 4, 5 - umgangssprachlich als "Bananenstecker" bezeichnet - , die über entsprechende Kabel elektrisch mit den Anschlussbuchsen eines nicht näher dargestellten Reizschwellenanalysators zu verbinden sind.

Der Adapter 1 weist einen im Wesentlichen quaderförmigen Grundkörper 6 auf, der aus einem durchsichtigen, sterilisierbarem Kunststoffmaterial in einem Stück spritzgegossen ist. Im Grundkörper ist etwa mittig zur Oberseite hin gerückt eine dazu parallel verlaufende, buchsenartige Aufnahme 7 angelegt, die mit ihrem zur Außenseite hin weisenden Querschnittsbereich in einem im Profil dreieck-förmigen Vorsprung 8 untergebracht ist. Dieser erstreckt sich über die Oberseite 9 des Adapters 1 hinweg.

Wie aus Fig. 1 deutlich wird, ist die Aufnahme 7 ausgehend von einem mündungsseitigen Steckbereich mit dem Durchmesser d10 zweifach abgestuft, nämlich auf einen etwas engeren Steckbereich 11 mit dem Durchmesser d11 und einem am tiefsten gelegenen Steckbereich 12 mit einem noch kleineren Durchmesser d12. Die Steckbereiche 10, 11 und 12 sind in ihren Durchmessern d10, d11 und d12 an die entsprechenden Außendurchmesser D13, D14, D15 des Steckerschaftes 13, des Ringkontaktes 14 und des an der Spitze des Steckers 2 gelegenen Spitzenkontaktes 15 angepasst. Der Schaft 13 ist dabei vom Ringkontakt 14 durch eine doppelte umlaufende Ringdichtung 16 abgetrennt. Desgleichen ist auf dem dem Ringkontakt 14 folgenden Schaftbereich vor der Abstufung zum Spitzenkontakt 15 ein weiteres Paar von Ringdichtungen 17 vorgesehen.

Zur Aufnahme der beiden Laborstecker 4, 5 weist der Adapter in zwei in Einsteckrichtung E hintereinander liegenden, dazu senkrechten Ebenen P18 und P19 je eine im Wesentlichen zylindrische Aufnahme 18, 19 auf. Wie aus Fig. 1 erkennbar ist, entsprechen diese Aufnahmen 18, 19 in der Tiefe etwa der Länge I der Laborstecker 4, 5 und enden kurz vor der der Mündung 20, 21 abgewandten Schmalseite 22 des Adapters 1. Dort sind sie über eine Öffnung 23, 24 jeweils für Sterilisationsflüssigkeit durchlässig.

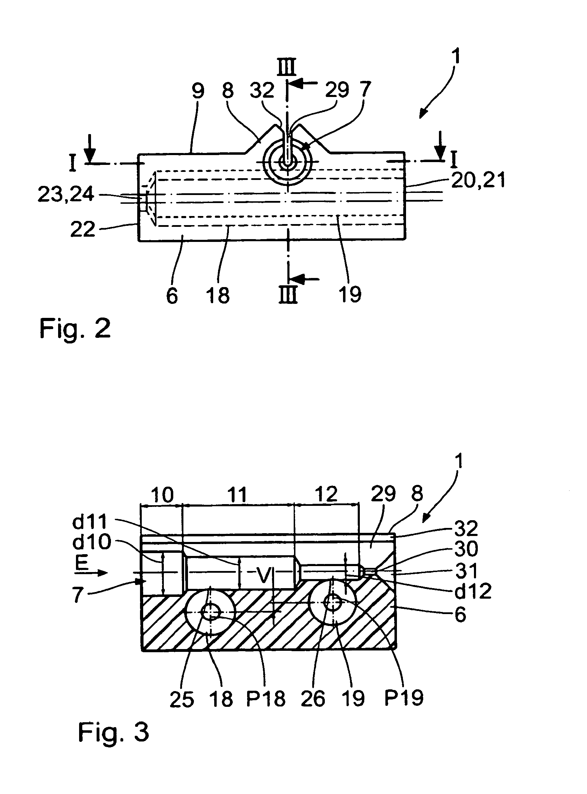

Wie insbesondere aus Fig. 3 deutlich wird, schneiden sich die Querschnitte der Aufnahme 7 für den IS-1 / IS-4 / DF-1-Stecker 2 einerseits und der beiden Aufnahmen 18, 19 für die Laborstecker 4, 5 andererseits knapp peripher, sodass die beiden Querschnittsvolumina über Kontaktfenster 25, 26 (Fig. 1) ineinander übergehen. Werden nun in die Aufnahme 7 der IS-1-Stecker 2 und in die Aufnahmen 18, 19 jeweils ein Laborstecker 4, 5 eingesteckt, so geben die Federlamellen 27 der Laborstecker 4, 5 über die Kontaktfenster 25, 26 jeweils eine optimale elektrische Verbindung zu dem Ringkontakt 14 bzw. Spitzenkontakt 15 des Steckers 2.

Wie besonders aus Fig. 3 deutlich wird, weisen die beiden Aufnahmen 18, 19 zur Anpassung an die stufenförmige Durchmesserverringerung der Aufnahme 7 einen Versatz V zueinander auf.

Für den oben erwähnten, in der Elektrode sitzenden Mandrin, der in Fig. 1 lediglich ausschnittsweise beim Bezugszeichen 28 angedeutet ist, ist die Aufnahme 7 für den Stecker 2 über ihre komplette Länge seitlich durch einen im Vorsprung 8 angelegten Durchführungsschlitz 29 offen. Ferner setzt sich deren innerster Steckbereich 12 in ein Durchführungsloch 30 in koaxialer Richtung fort, das sich zu einer trichterförmigen Mündung 31 weitet. Über den Durchführungsschlitz 29 und das Durchführungsloch 30 ist der vor dem Spitzenkontakt 15 laufenden Abschnitt des Mandrins 28 in die Aufnahme 7 von der Seite her einführbar. Ein direktes Einfädeln ist nicht möglich, da der Mandrin in einem voluminösen Steuergriff endet. Nach dem Einführen des Mandrins in die Aufnahme 7 kann der Stecker 2 dann eingesteckt werden. Zur Erleichterung der Mandrin-Einführung ist der Durchführungsschlitz 29 mit Einführschrägen 32 versehen.

## Patentansprüche

1. Kontaktverbindungs-Adapter zur Herstellung eines zeitweisen elektrischen Kontaktes zwischen einem ersten Stecker (2), insbesondere einem Norm-Anschluss-Stecker einer Herzschrittmacher-Elekrode (3), und mindestens einem weiteren Stecker (4, 5), insbesondere einem üblichen Laborstecker eines Laborkabels zur zeitweisen Verbindung der Elektrode (3) mit einem Reizschwellenanalysator, der Kontaktverbindungs-Adapter umfasst
- einen Grundkörper (6),
- eine erste im Grundkörper (6) angelegte, buchsenartige Aufnahme (7) für den einen Stecker (2), und
- mindestens eine zweite im Grundkörper (6) angelegte, buchsenartige Aufnahme (18, 19) für den mindestens einen weiteren Stecker (4, 5),
**dadurch gekennzeichnet, dass** sich die Querschnitte der ersten und der mindestens einen zweiten Aufnahme (7, 18, 19) derart peripher schneiden, dass in die Aufnahmen (7, 18, 19) eingesteckte Stecker (2, 4, 5) an ihren seitlichen Kontaktflächen (14, 15, 27) aneinander anliegen und einen elektrischen Kontakt herstellen.

2. Kontaktverbindungs-Adapter nach Anspruch 1, **dadurch gekennzeichnet, dass** die Aufnahmen (7, 18, 19) für die Stecker (2, 4, 5) mit ihren Längsachsen in einander senkrecht schneidenden Ebenen (P7, P18, P19) angeordnet sind.

3. Kontaktverbindungs-Adapter nach Anspruch 1 oder 2, **dadurch gekennzeichnet, dass** für einen bipolaren oder unipolaren Stecker (2) eine mit zunehmender Buchsentiefe zweifach im Durchmesser (d11, d12) abgestufte Aufnahme (7) zur deren Anpassung an die stufig abnehmenden Durchmesser (D13, D14, D15) von Steckerschaft (13), Ringkontakt (14) und Spitzenkontakt (15) des Steckers (2) vorgesehen ist.

4. Kontaktverbindungs-Adapter nach Anspruch 3, **dadurch gekennzeichnet, dass** zwei parallel nebeneinander liegende Aufnahmen (18, 19) für zwei weitere Stecker (4, 5) im Grundkörper (6) angelegt sind, die einen Versatz (V) zueinander entsprechend der Durchmesserverringerung zwischen Ring- (14) und Spitzenkontakt (15) des bipolaren Steckers (2) aufweisen.

5. Kontaktverbindungs-Adapter nach einem der vorgenannten Ansprüche, **dadurch gekennzeichnet, dass** das die Aufnahme (7) für den ersten Stecker (2) sowohl am tiefen Ende in längsachsialer Richtung über eine dünne Durchführung (30) als auch entlang ihrer gesamten Tiefe über einen seitlichen, längsparallelen Durchführungsschlitz (29) offen ist.

6. Kontaktverbindungs-Adapter nach Anspruch 5, **dadurch gekennzeichnet, dass** der Durchführungsschlitz (29) mit Einführschrägen (32) versehen ist.

7. Kontaktverbindungs-Adapter nach einem der vorgenannten Ansprüche, **dadurch gekennzeichnet, dass** die mindestens eine Aufnahme (18, 19) für den mindestens einen weiteren Stecker (4, 5) am tiefen Ende ein Öffnung (23, 24) aufweist.

8. Kontaktverbindungs-Adapter nach einem der vorgenannten Ansprüche, **dadurch gekennzeichnet, dass** der Grundkörper (6) aus einem durchsichtigen, sterilisierbaren Kunststoffmaterial besteht.

## Claims

1. A contact connection adapter for producing a temporary electrical contact between a first plug (2), in particular a standard terminal plug of a cardiac pacemaker electrode (3), and at least one further plug (4, 5), in particular a typical laboratory plug of the laboratory cable for temporary connection of the electrode (3) to a stimulus threshold analyzer, the contact connection adapter comprising
- a main body (6),
- a first socket-like receptacle (7), which is placed in the main body (6), for the one plug (2), and
- at least one second socket-like receptacle (18, 19), placed in the main body (6), for the at least one further plug (4, 5),
**characterized in that** the cross-sections of the first and the at least one second receptacle (7, 18, 19) peripherally intersect in such a way that plugs (2, 4, 5) inserted into the receptacles (7, 18, 19) fit tightly one another at their lateral contact surfaces (14, 15, 27) and produce an electrical contact.

2. The contact connection adapter according to Claim 1, **characterized in that** the receptacles (7, 18, 19) for the plugs (2, 4, 5) are situated having their longitudinal axes in intersecting planes (P7, P18, P19) perpendicular to one another.

3. The contact connection adapter according to Claim 1 or 2, **characterized in that**, for a bipolar or unipolar plug (2), a receptacle (7) which is stepped twice in diameter (d11, d12) with increasing socket depth, is provided for its adaptation to the stepped, decreasing diameters (D13, D14, D15) of plug shaft (13), ring contact (14), and tip contact (15) of the plug (2).

4. The contact connection adapter according to Claim 3, **characterized in that** two receptacles (18, 19) lying parallel neighboring one another for two further plugs (4, 5) are placed in the main body (6), which have an offset (V) to one another corresponding to the diameter reduction between ring contact (14) and tip contact (15) of the bipolar plug (2).

5. The contact connection adapter according to one of the preceding claims, **characterized in that** the receptacle (7) for the first plug (2) is open both at the deep end in the longitudinally-axial direction via a thin passage (30) and also along its entire depth via a lateral, longitudinally-parallel passage slot (29).

6. The contact connection adapter according to Claim 5, **characterized in that** the passage slot (29) is provided with insertion bevels (32).

7. The contact connection adapter according to one of the preceding claims, **characterized in that** at least one receptacle (18, 19) has an opening (23, 24) at the deep end for the at least one further plug (4, 5).

8. The contact connection adapter according to one of the preceding claims, **characterized in that** the main body (6) comprises a transparent plastic material which may be sterilized.

## Revendications

1. Adaptateur pour liaison de contact pour l'établissement d'un contact électrique temporaire entre une première fiche (2), en particulier une fiche de raccordement normalisée d'une électrode de pacemaker (3) et au moins une autre fiche (4, 5), en particulier une fiche de laboratoire classique d'un câble de laboratoire pour la liaison de l'électrode (3) avec un analyseur de seuil d'irritation, lequel adaptateur de liaison de contact comprend
- un corps de base (6),
- un premier logement (7) de type douille placé dans le corps de base(6) pour la première fiche (2), et
- au moins un second logement (18, 19) de type douille placé dans le corps de base (6) pour la au moins une autre fiche (4, 5), **caractérisé en ce que** les sections du premier et du au moins un second logement (7, 18, 19) se coupent à la périphérie de telle sorte que des fiches (2, 4, 5) enfichées dans les logements (7, 18, 19) s'appliquent les unes sur les autres sur leurs surfaces de contact (14, 15, 27) latérales et établissent un contact électrique.

2. Adaptateur pour liaison de contact selon la revendication 1, **caractérisé en ce que** les logements (7, 18, 19) pour les fiches (2, 4, 5) sont disposés avec leurs axes longitudinaux dans des plans (P7, P18, P19) se coupant à la perpendiculaire.

3. Adaptateur pour liaison de contact selon la revendication 1 ou 2, **caractérisé en ce que**, pour une fiche (2) bipolaire ou unipolaire, il est prévu un logement (7) à double dégradé en diamètre (d11, d12) avec la profondeur de douille croissante pour son adaptation aux diamètres (D13, D14, D15) décroissant en dégradé de la tige de fiche (13), du contact annulaire (14) et du contact de sommet (15) de la fiche (2).

4. Adaptateur pour liaison de contact selon la revendication 3, **caractérisé en ce que** deux logements (18, 19) disposés parallèlement l'un à côté de l'autre destinés à deux autres fiches (4, 5) sont placés dans le corps de base (6), lesquels présentent un déport (V) l'un par rapport à l'autre conformément à la réduction de diamètre entre le contact annulaire (14) et le contact de sommet (15) de la fiche (2) bipolaire.

5. Adaptateur pour liaison de contact selon l'une quelconque des revendications précédentes, **caractérisé en ce que** le logement (7) pour la première fiche (2) est ouvert aussi bien sur l'extrémité profonde dans la direction de l'axe longitudinal par un passage (30) mince que le long de sa profondeur globale par une fente de passage (29) latérale et parallèle dans la longueur.

6. Adaptateur pour liaison de contact selon la revendication 5, **caractérisé en ce que** la fente de passage (24) est dotée de chanfreins d'introduction (32).

7. Adaptateur pour liaison de contact selon l'une quelconque des revendications précédentes, **caractérisé en ce que** le au moins un logement (18, 19) présente une ouverture (23, 24) sur l'extrémité profonde pour la au moins une autre fiche (4, 5).

8. Adaptateur pour liaison de contact selon l'une quelconque des revendications précédentes, **caractérisé en ce que** le corps de base (6) est à base d'un matériau plastique transparent et stérilisable.
